Europäisches Patentamt

European Patent Office

Office européen des brevets

(19)

(11) Publication number: **0 025 348**
**A1**

## (12) EUROPEAN PATENT APPLICATION

(21) Application number: **80303120.2**

(22) Date of filing: **05.09.80**

(51) Int. Cl.³: **C 07 C 79/12**
**C 07 C 76/02**

(30) Priority: **07.09.79 US 73405**

(43) Date of publication of application:
**18.03.81 Bulletin 81/11**

(84) Designated Contracting States:
**DE FR GB IT**

(71) Applicant: **MONSANTO COMPANY**
**Patent Department 800 North Lindbergh Boulevard**
**St. Louis, Missouri 63166(US)**

(72) Inventor: **Schumacher, Ignatius**
**336 West Clayton Road**
**Ballwin Missouri 63011(US)**

(74) Representative: **Pearson, John Lionel et al,**
**Monsanto House 10-18 Victoria Street**
**London, SW1H ONQ(GB)**

(54) **A process for the nitration of halobenzenes.**

(57) Para isomer formation of nitrohalobenzene is obtained by conducting the nitration of halobenzene in the presence of mixtures of methanesulfonic acid and sulfuric acid.

EP 0 025 348 A1

Croydon Printing Company Ltd.

-1-

A PROCESS FOR THE             C23-54-0080
NITRATION OF HALOBENZENES
BACKGROUND OF THE INVENTION

This invention relates to the preparation of nitrohalobenzenes by the nitration of the corresponding halobenzene and particularly to obtaining a para isomer directive effect.

U.S. Patent No. 3,077,502 teaches that the isomer distribution in the nitrohalobenzene product is influenced when the nitration of a halobenzene compound is carried out in the presence of a sulfonic acid. The presence of even small amounts of a sulfonic acid markedly influences the isomer distribution in a nitrohalobenzene product and this effect is still obtained when a high concentration of sulfonic acid is employed, i.e., where 3 moles of sulfonic acid per mole of halobenzene are used. Moreover, the amount of the desired isomer obtained when the nitration is carried out in the presence of a sulfonic acid is greater than the amount obtained in the absence of a sulfonic acid regardless of the nitrating agent or nitrating temperature employed. This patent discloses sulfonic acids, i.e., those containing one or more sulfonic acid groups and equates the applicability of all sulfonic acids whether aliphatic, cycloaliphatic, aromatic and heterocyclic.

The para isomer of nitrohalobenzene is in greater demand today than the ortho isomer and although the above-noted patent provides for increasing the para isomer content, further increase in the para isomer over the ortho isomer would offer advantages to the industry.

-2-                                        0025348

Nitrochlorobenzene is prepared, as described in U.S. Patent No. 3,077,502 by reacting concentrated nitric acid with monochlorobenzene in the presence of a mixture of a major portion of a sulfonic acid and a minor portion of sulfuric acid, the quantity of sulfonic acid added being within the range of from about 0.5 to about 2 moles per mole of monochlorobenzene added. It is preferred that the mixture contain at least about 1.5 parts by weight of sulfonic acid per part of sulfuric acid. It is even more preferred to use a mixture which contains at least 3.0 parts of sulfonic acid per part by weight of sulfuric acid. Expressed on a percentage basis the concentration of the two compounds should be within the range of from about 60% to 90% of sulfonic acid and correspondingly from 40% to 10% sulfuric acid. Such mixtures are desirably obtained from a process for the sulfonation of benzene employing 25-35% oleum, wherein a mixture containing approximately 72% by weight of benzene sulfonic acid, 22% sulfuric acid, 5% water, and 1% of a mixture of sulfones, benzene and benzenedisulfonic acid is obtained.

## SUMMARY

This invention is directed to the use of methanesulfonic acid as the sulfonic acid in the nitration of halobenzenes.

Typical objects of this invention are (1) to provide an improvement in the nitration of halobenzene in order to increase the para isomer formation and (2) to provide a process for the nitration of halobenzene directive to the para isomer through the use of methanesulfonic acid.

According to this invention, increased para isomer formation of nitrohalobenzene is obtained by conducting the nitration of halobenzene with a nitrating agent in the presence of a mixture of methanesulfonic acid and sulfuric acid.

The halobenzenes which can be employed in the process of this invention include monochlorobenzene as well as the other monohalogenated benzenes, e.g., monobromobenzene, monoiodobenzene and monofluorobenzene.

Any nitrating agent which is capable of effecting the nitration of an aromatic ring can be used in the process of this invention, e.g., mixed acid, concentrated nitric acid, nitric anhydride, nitrogen tetraoxide, ethyl nitrate, etc. Generally, the nitrating agent is employed in stoichiometric quantities, or slightly in excess of the amount required to effect the mononitration of the monohalobenzene. Concentrated nitric acid, which contains 90% or more by weight of $HNO_3$ is a preferred nitrating agent.

The aforementioned methanesulfonic acid may be further substituted by one or more substituents. Typical but not limitative of such substituents are the halogens, such as chlorine, bromine, iodine and fluorine; carboxy and other non-reactive substituents. Illustrative examples of such are chloromethane sulfonic acid and trichloromethane sulfonic acid.

In cases where the nitrohalobenzene product crystallizes from the reaction medium it may be advantageous to employ an excess of the halobenzene reactant to act as a solvent, or another solvent may be employed, such as a petroleum ether; a saturated aliphatic hydrocarbon, such as hexane, octane, cyclohexane, etc.; or a liquid chlorinated hydrocarbon such as chloroform, carbon tetrachloride or tetrachloroethane.

Effective concentrations of the methanesulfonic acid in the process of this invention vary in wide ranges depending upon the result desired in that extremely low amounts, e.g., 0.05 mole of methanesulfonic acid per mole of halobenzene to be nitrated will produce the para directive effect. Higher yields and higher para isomer content are achieved when the concentration of methanesulfonic acid is within the range of from about 0.5 to about 2 moles per mole of halobenzene to be nitrated. It is even more preferred to employ from about 1.0 to about 2.0 moles of methanesulfonic acid per mole of halobenzene to be nitrated. Amounts greater than three moles of methanesulfonic acid per mole of halobenzene to be nitrated can be used, however no significant increase in either para isomer content or yield results.

Those skilled in the art will recognize that the process of this invention is not limited to specific reaction temperatures, since the process can be carried out at temperatures of from -30 to temperatures of 160°C. or more. A reaction temperature of -30°C. can be maintained, for example, by employing a cooling bath comprising a slurry of solid carbon dioxide in acetone and using chloroform as a reaction diluent. As will be appreciated, the rate of reaction at temperatures of from -30°C. to 0°C. will be somewhat slow. The minimum temperature for the process of this invention is therefore that temperature just above that at which no reaction between the nitrating agent and the halobenzene will take place. The maximum temperature is only of economic importance, for it is dependent on economic factors rather than technical factors. Temperatures within the range of from about 30°C. to about 90°C. are desirably used, while temperatures within the range of from 50°C. to 60°C. are especially preferred.

After the nitration reaction is complete, the product can be recovered from the reaction by any method well known to those skilled in the art. For example, the reaction mixture is permitted to settle into two phases, i.e., an organic phase and an acid phase. The organic phase is then separated and the acid phase is cooled to precipitate the product and the product is removed by filtration. This method can be varied by employing a solvent extraction technique to remove the residual product from the acid phase. Preferably, the acid portion is extracted with halobenzene which is then recycled to the reaction and the remaining acid is dehydrated for reuse.

## EXAMPLES

The manner of carrying out the process of this invention and the improvement if offers over U.S. Patent No. 3,077,502 will be further apparent from the following specific examples in which all parts are parts by weight unless otherwise specified.

## EXAMPLE 1

To a well stirred mixture of 176 parts of benzenesulfonic acid monohydrate and 100 parts of 98% sulfuric acid (60/40 ratio) and 123 parts of chlorobenzene there is slowly added 64 parts of 98% nitric acid. The temperature is maintained at 45-50°C. by cooling and the nitric acid is added over a 45 minute period. The reaction mixture is maintained at 70-75°C. for 30 minutes. The resulting mixture is then diluted with 200 parts of water, permitted to separate into two phases and the bottom layer is drawn off. The product oil is washed at 70-80°C. with two aliquots containing 200 parts by weight of a 5% sodium carbonate solution and finally with two additional aliquots of water. The washed oil is then heated at reduced pressure to remove the residual water and any unreacted chlorobenzene. Analysis of the product shows a 92% yield of nitrochlorobenzene with about 68% of the para isomer and about 32% of the ortho isomer for a p/o ratio of about 2.12.

## EXAMPLE 2

Example 1 is repeated except that the mixture contained 105 parts of benzenesulfonic acid monohydrate and 123 parts of chlorobenzene and 120 parts of 85% sulfuric acid and 65 parts of 90% nitric acid were mixed and slowly added over a 45 minute period. The sulfonic/sulfuric ratio is 50/50.

Analysis of the product shows a 71% yield of nitrochlorobenzene with about 47% of the para isomer and about 24% of the ortho isomer for a p/o ratio of about 1.96.

### EXAMPLE 3

Example 1 is repeated except that 90 parts of methanesulfonic acid and 60 parts of sulfuric acid (60/40 ratio) is used. Analysis of the product shows a 99% yield of nitrochlorobenzene with about 65.8% of the para isomer and about 30.5% of the ortho isomer for a p/o ratio of about 2.25.

### EXAMPLE 4

Example 1 is repeated except that 80 parts of methanesulfonic acid and 80 parts of sulfuric acid (50/50 ratio) is used. Analysis of the product shows a 99% yield of nitrochlorobenzene with about 68% of the para isomer and 31% of the ortho isomer for a p/o ratio of about 2.20.

Comparison of the para/ortho ratios obtained in the above Examples using benzenesulfonic acid and methanesulfonic acid is shown in the following Table:

| 60/40 Acid Ratio | P/O Ratio |
|---|---|
| benzenesulfonic/sulfuric | 2.12 |
| methanesulfonic/sulfuric | 2.25 |
| 50/50 Acid Ratio | |
| benzenesulfonic/sulfuric | 1.96 |
| methanesulfonic/sulfuric | 2.20 |

From this comparison it is readily apparent that the improvement of this invention, i.e., use of methanesulfonic acid, results in about 5 to 10% improvement over use of other sulfonic acids in the para isomer direction.

In addition to the above-noted para isomer yield improvement by practice of this invention, another commercial attractive advantage of this invention is the

**0025348**

freedom for continuous dehydration and recycle of the methanesulfonic and sulfuric acid mixture in the production of nitrohalobenzene. Benzenesulfonic acid forms with nitric acid nitrobenzenesulfonic acid which at ambient temperature forms crystals and must be purged from the system.

While this invention has been described with respect to certain embodiments, it is not so limited and it is to be understood that variations and modifications thereof which are obvious to those skilled in the art may be made without departing from the spirit or scope of this invention.

WHAT IS CLAIMED IS:

1. A process for the nitration of halobenzenes, wherein the reaction is carried out in the presence of a sulfonic acid in an amount which is sufficient to produce a para directive effect, characterized in that the sulfonic acid is a methanesulfonic acid.

2. The process of Claim 1 characterized in that said nitration of halobenzene is by the nitric acid nitration of the corresponding halobenzene.

3. The process of Claim 2 characterized in that the halobenzene is monochlorobenzene.

4. The process of Claim 1 for the production of nitrochlorobenzene characterized by reacting monochlorobenzene with concentrated nitric acid at a temperature within the range of from about $0^{o}C.$ to about $160^{o}C.$ in the presence of a mixture comprising a major portion of a methanesulfonic acid and a minor portion of sulfuric acid, the quantity of sulfonic acid added being from about 0.5 mole to about 2 moles per mole of chlorobenzene.

5. The process of Claim 4 characterized in that the concentrated nitric acid is present in at least a stoichiometric quantity.

6. The process of Claim 4 characterized in that the temperature is within the range of $30^{o}C.$ to $90^{o}C.$

7. The process of Claim 1 characterized in that 1 to 2 moles of methanesulfonic acid per mole of halobenzene is present.

| Category | Citation of document with indication, where appropriate, of relevant passages | Relevant to claim |
|---|---|---|
| | US - A - 3 180 900 (ALLEN K. SPARKS) <br> + Claim 1; example IV + | 1,3 |
| | US - A - 3 253 045 (ALLEN K. SPARKS) <br> + Claim 1; example III + | 1,3 |
| A | US - A - 3 183 275 (ALLEN K. SPARKS) <br> + Claim 1 + | 1 |
| | US - A - 3 140 319 (ALLEN K. SPARKS) <br> + Claim 1; example I + | 1,3 |
| | GB - A - 1 484 589 (TEIJIN) <br> + Claims 1-3; example 1 + | 1-3 |
| | US - A - 3 928 476 (KEIZO SHIMADA) <br> + Claim 1; examples 1-7 + | 1-3,5 |
| | US - A - 3 153 674 (DAVID E. DUNLAP) <br> + Claim 1 + | 1,3 |
| | US - A - 4 107 220 (DENNIS C. OWSLEY) <br> + Claims 1,2 + | 1,3 |

**DOCUMENTS CONSIDERED TO BE RELEVANT**

**CLASSIFICATION OF THE APPLICATION (Int. Cl. 3)**

C 07 C 79/12
C 07 C 76/02

**TECHNICAL FIELDS SEARCHED (Int.Cl. 3)**

C 07 C 79/00
C 07 C 76/00

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X

The present search report has been drawn up for all claims

| Place of search VIENNA | Date of completion of the search 13-11-1980 | Examiner REIF |
|---|---|---|

EPO Form 1503.1 06.78